# EUROPEAN PATENT APPLICATION

(11) **EP 3 939 619 A1**
(43) Date of publication of application: **19.01.2022**
(21) Application number: 21185674.5
(22) Date of filing: 14.07.2021
(51) Int. Cl.: A61L 2/00, A61L 2/24

(54) **DISINFECTION ASSEMBLY**

(30) Priority: 14.07.2020 IN 202041029938
(71) Applicant: Goodrich Aerospace Services Private Limited, 560048 Bengaluru (IN)
(72) Inventor: TALAKADU, Madhu, 570019 Mysuru (IN)
(74) Representative: Dehns

(57) **Abstract**

A disinfection assembly for a person includes a chamber housing (102) defining a floor area (104), an entry (106), an exit (108) and an interior space (110) between the entry and the exit. At least one of a UV light emitter (314) or an atomization nozzle (114) is positioned within the interior space. At least one platform (112) is positioned and a sensor (116) operatively coupled to the platform. A method for disinfecting a person in a disinfection assembly includes triggering at least one sensor in a disinfection chamber housing. The method includes at least one of emitting a UV light with a UV light emitter, or spraying a disinfection fluid from an atomization nozzle for a stipulated duration. The method includes stopping at least one of the emitting or the spraying after the stipulated duration. The method includes opening an exit door to permit a person to exit the disinfection chamber housing after the stipulated duration.

## Description

### CROSS-REFERENCE TO RELATED APPLICATIONS

This application claims priority to and the benefit of Indian Application No. 202041029938, filed July 14, 2020.

### BACKGROUND

### 1. Field

The present disclosure relates to disinfection assemblies, and more particularly disinfection chambers to be used in airports and on aircrafts.

### 2. Description of Related Art

As a result of the COVID-19 pandemic there is a need for better awareness and methods of disinfection to prevent the spread of COVID-19 and other pathogens during travel, e.g. air travel, rail travel or the like. The new landscape of the world, post-pandemic may generate a new normal where improving sanitation and cleanliness, both in a terminal, for example, and on-board, for both the passengers and crew is of the utmost importance in order to ensure healthy and safe travel conditions for the future of commercial transportation. This is especially true, when considering crowded areas that have masses of people passing through them on a daily basis, such as airports and on aircrafts. Traditional disinfection chambers have been used in hospitals and medical industries for disinfecting equipment, or the like.

The conventional techniques have been considered satisfactory for their intended purpose. However, there is an ever present need for improved systems and methods for off-board and on-board passenger disinfection.

### SUMMARY

A disinfection assembly for a person includes a chamber housing defining a floor area, an entry, an exit and an interior space between the entry and the exit. At least one of a UV light emitter or an atomization nozzle is positioned within the interior space. At least one platform is positioned and a sensor operatively coupled to the platform.

The UV light emitter can be configured and adapted to be turned on when a passenger is sensed on the platform. The UV light emitter can emit light having a wavelength that ranges from 200 nm to 280 nm. The UV light emitter can include a plurality of UV light emitters. A heat sink is in thermal communication with the UV light to dissipate heat therefrom. The atomization nozzle is configured and adapted to be in fluid communication with a disinfection fluid tank. A pump can be mounted above the disinfection fluid tank. The atomization nozzle can be configured and adapted to be turned on when a passenger is sensed on the platform. The atomization nozzle can include a plurality of atomization nozzles. Each of the plurality of atomization nozzles can be mounted to the chamber housing.

A method for disinfecting a person in a disinfection assembly includes triggering at least one sensor in a disinfection chamber housing. The method includes emitting a UV light with a UV light emitter, and/or spraying a disinfection fluid from an atomization nozzle for a stipulated duration. The method includes stopping at least one of the emitting or the spraying after the stipulated duration. The method includes opening an exit door to permit a person to exit the disinfection chamber housing after the stipulated duration.

The method can include opening an entry door to permit a person to enter the disinfection chamber housing. The opening of the entry door and/or opening of the exit door can be automatic. Spraying the disinfection fluid includes starting a pump mounted above a disinfectant fluid tank. Stopping the spraying includes turning off a pump mounted above a disinfectant fluid tank. Emitting a UV light with the UV light emitter includes a emitting UV light with a plurality of UV light emitters. Emitting a UV light with the UV light emitter includes emitting light having a wavelength that ranges from 200 to 280 nm. The atomization nozzle includes a plurality of atomization nozzle, wherein each of the plurality of atomization nozzles is mounted to the chamber housing. The method can include dissipating heat away from the UV light emitter with a heat sink.

These and other features of the systems and methods of the subject disclosure will become more readily apparent to those skilled in the art from the following detailed description of the preferred embodiments taken in conjunction with the drawings.

### BRIEF DESCRIPTION OF THE DRAWINGS

So that those skilled in the art to which the subject disclosure appertains will readily understand how to make and use the devices and methods of the subject disclosure without undue experimentation, preferred embodiments thereof will be described in detail herein below with reference to certain figures, wherein:
Fig. 1 is a schematic perspective view of a disinfection chamber assembly constructed in accordance with embodiments of the present disclosure, showing a plurality of nozzles;
Fig. 2 is a schematic perspective view of another disinfection chamber assembly constructed in accordance with embodiments of the present disclosure, showing a plurality of UV light emitters; and
Fig. 3 is a schematic perspective view of another disinfection chamber assembly constructed in accordance with embodiments of the present disclosure, showing a plurality of UV light emitters.

### DETAILED DESCRIPTION

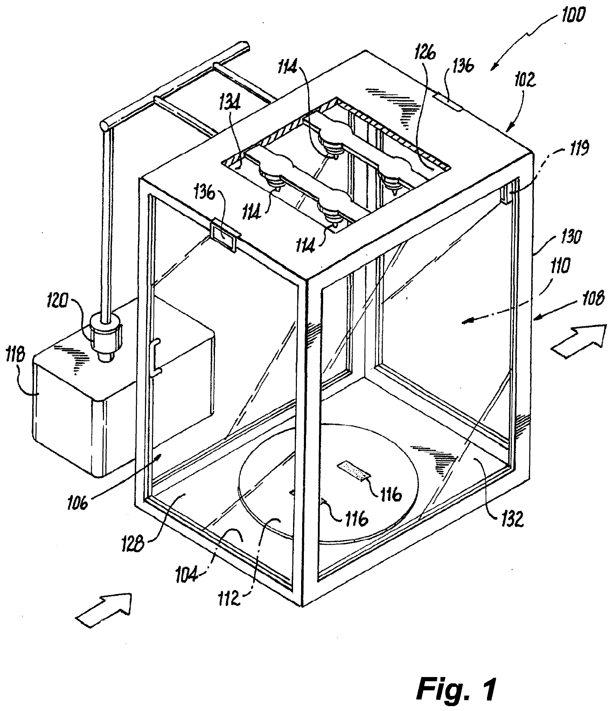

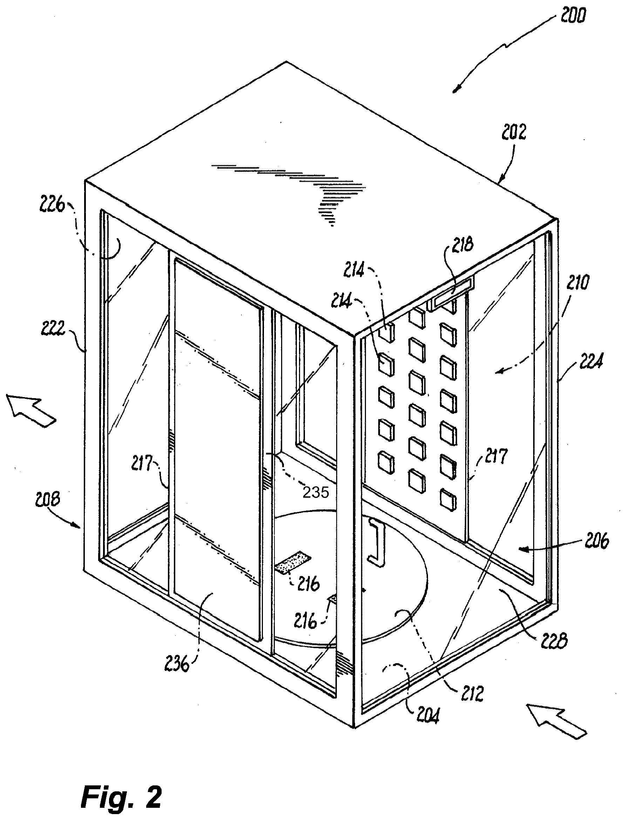

Reference will now be made to the drawings wherein like reference numerals identify similar structural features or aspects of the subject disclosure. For purposes of explanation and illustration, and not limitation, a partial view of an embodiment of a disinfection assembly in accordance with the disclosure is shown in Fig. 1 and is designated generally by reference character 100. Other embodiments of systems in accordance with the disclosure, or aspects thereof, are provided in Figs. 2-3 as will be described. The systems and methods described herein provide multiple options for disinfection. From airport to inside the aircraft, embodiments of the disinfection chamber assemblies of the present disclosure provide disinfection facilities to passengers by killing virus on the surface of their fabric clothing or their personal protective equipment, e.g. "PPE" (Face shield, gowns, covers etc.).

As shown in Fig. 1, a disinfection assembly 100, e.g. a "flash" disinfection assembly, for a person includes a chamber housing 102 defining a floor area 104, an entry 106, an exit 108 and an interior space 110 between the entry 106 and the exit 108. Chamber housing 102 includes a metal frame 130 and transparent acrylic side walls 132 and 134. COVID-19 virus may be on the surface of fabric or PPE that passengers are wearing. This may result in transmission if touched. According to CDC (Centers for Disease Control & Prevention) the virus can survive up to 24 hours on fabric surface and on plastic, stainless steel for up to two days. Disinfection assembly 100 provides a way to disinfect PPE that is being worn by passengers so that transmission through touch can be reduced or eliminated. A platform 112 is positioned in the interior space 110.

As shown in Fig. 1, a plurality of atomization nozzles 114 are positioned within the interior space 110 and are mounted to the chamber housing 102 around an upper portion of the housing. Nozzles 114 are configured and adapted to spray disinfectant chemicals into the interior space 110. The disinfectant chemical can be an alcohol based chemical disinfectant stored in a disinfection fluid tank 118. Because of the flammable nature of the chemical disinfectant, assembly 100 tends to be suited for in-terminal disinfection for passengers wearing PPE. Sensors 116 are operatively coupled to the platform 112. While a plurality of sensors 116 are shown, those skilled in the art will readily appreciate that a single sensor, such as a strain-gauge based load cell or the like, could be used. Disinfection assembly 100 could be used prior to boarding the plane, train, etc., for example, in a terminal or at security checkpoint.

With continued reference to Fig. 1, each atomization nozzle 114 is configured and adapted to be in fluid communication with disinfection fluid tank 118. The tank 118 is kept close to disinfectant chamber. A pump 120 is mounted above the disinfection fluid tank 118 to draw fluid from the tank to the nozzles 114. Each atomization nozzle 114 is configured and adapted to be directed toward the platform 112 and the area above the platform 112. Assembly 100 includes an entrance door 128 at entry 106 to contain the spraying during the disinfection. Door 106 is configured and adapted to open automatically using sensors 136 in order to permit a person to enter the disinfection chamber and then closes once sensor 116 is triggered. Once the passenger enters and stands on platform 112, the sensor 116 sends signals to turn on the pump 120 mounted above the chemical disinfectant tank 118. The pump 120 are turned on when a passenger is sensed on the platform 112 by the sensor, thereby starting fluid flow to atomization nozzles 114. Once on, pump 120 is configured and adapted to run for a duration of time, then shut-off, thereby stopping fluid flow through nozzles 114. Assembly 100 includes an exit door 126 at exit 108 to contain the spraying during the disinfection. Door 126 is configured and adapted to open automatically, for example by triggering another sensor 136 at exit 108, in order to permit a person to exit the disinfection chamber after the spraying has stopped. It is also contemplated that door 126 can be automatically opened based on timing after the disinfection spray has stopped. It is also contemplated that a green light or other indicator 119 can be used to signal to the passenger that the disinfection process has completed. Flash type disinfection chambers, e.g. assembly 100, are designed to use inside the airport or other common terminal. Assembly 100 can be placed at the terminal gate where passengers board the flight after thermal scanning and a health assessment. It is also contemplated that UV light disinfection chambers, e.g. like disinfection chamber assembly 200, described below, can also be used in same location, but separate from the disinfection chamber 100 (for safety reasons). After the disinfection passengers can enter the aircraft through a passenger boarding bridge.

With reference now to Fig. 2, a disinfection chamber assembly 200 for a person includes a chamber housing 202 defining a floor area 204, an entry 206, an exit 208 and an interior space 210 between the entry 206 and the exit 208. Assembly 200 includes an exit door 226 at exit 208 to contain the UV light during the disinfection. Assembly 100 includes an entrance door 228 at entry 206 to contain the UV light during the disinfection. Doors 226 and 228 are configured and adapted to open automatically to allow a person to enter the chamber or exit the disinfection chamber after the UV light emission has stopped. Doors at entry 206 and/or exit 208 are configured and adapted to be opened automatically using sensors 218. A plurality of UV light emitters 214 are positioned within the interior space 210. Assembly 200 is suitable for use in airports. A platform 212 is positioned in the interior space 210. Sensors 216 are operatively coupled to the platform 212. While a plurality of sensors 216 are shown, those skilled in the art will readily appreciate that a single sensor, such as a strain-gauge based load cell or the like, could be used.

With continued reference to Fig. 2, each UV light emitter 214 is configured and adapted to be turned on when a passenger is sensed on the platform 212. Once on, UV light emitters 214 are configured and adapted to run for a duration of time, for example, 10 seconds, then shut-off Each UV light emitter 214 is configured and adapted to emit light having a wavelength that ranges from 200 nm to 280 nm, which is safe for persons to be exposed to. UV light at this wavelength generally can destroy COVID-19 and other pathogens within 5 to 10 seconds, while still being invisible to human eyes and safe to use. The UV light emitters 214 are arranged on a panel 217. The assembly 200 includes two panels 217, one on a first side 222 of chamber housing 202 and one on a second side 224 of chamber housing 202. The UV light emitters can be UVC LEDs. Assembly 200 includes respective heat sinks 236 in thermal communication with each UV light emitters 214 to dissipate heat therefrom for safe operation. Each heat sink 236 is mounted on or proximate to an outer side 235 of each panel 217. Heat sinks 236 are constructed from aluminium or other suitable material. Assembly 200 offers health advantages overs some other types of disinfection as it only requires exposure to UV light during entry and exit. UV lights are energy efficient and they reduce disinfection time relative to other disinfection methods.

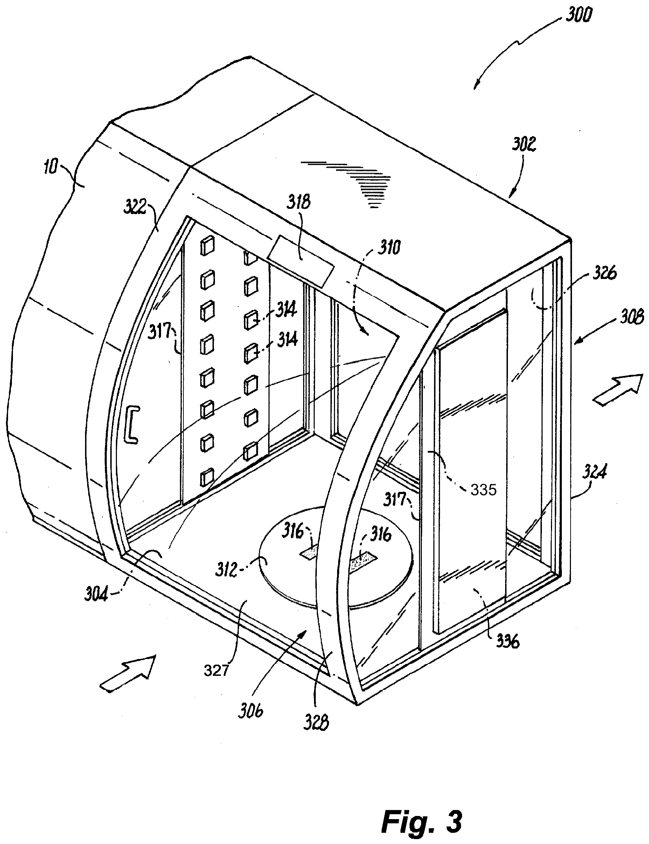

As shown in Fig. 3, another embodiment of a disinfection chamber assembly 300 for a person includes a chamber housing 302 defining a floor area 304, an entry 306, an exit 308 and an interior space 310 between the entry 306 and the exit 308. Assembly 300 is similar to assembly 200, except that assembly 300 is defined by a chamber housing 302 configured and adapted to be positioned in a fuselage 10 of an aircraft. By integrating assembly 300 into a fuselage 10, the airliner, or other common carrier will have autonomy over its disinfection protocol and it would not have to rely on local airport authority. The curvature section 328 of chamber housing 302 will be matched to fuselage section 10 at the aircraft entrance. Chamber assembly 300 can be fitted using clamping arrangements to aircraft frame. Since the chamber is fitted inside the aircraft there might be separate powers supply arrangements required to operate the lights inside the chambers.

With continued reference to Fig. 3, assembly 300 includes an exit door 326 at exit 308 to contain the UV light during the disinfection. Assembly 300 includes an entrance door 327 at entry 306 to contain the UV light during the disinfection. Doors 327 and 326 are configured and adapted to open automatically to allow a person to enter the chamber or exit the disinfection chamber after the UV light emission has stopped. Doors at entry 306 and/or exit 308 are automatically operated using sensor 318. It is contemplated that doors 327 or 326 can slide between open and closed positions. A platform 312 is positioned in the interior space 310. Sensors 316 are operatively coupled to the platform 312 and are similar to sensors 212. A plurality of UV light emitters 314, similar to UV light emitters 214, are positioned within the interior space 310. The UV light emitters 314 are arranged on panels 317, similar to panels 217. The assembly 300 includes two panels 317, one on a first side 322 of chamber housing 302 and one on a second side 324 of chamber housing 302. Assembly 300 includes respective heat sinks 336 in thermal communication with each UV light emitters 314 to dissipate heat therefrom for safe operation. Heat sinks 336 are constructed from aluminium or other suitable material. Each heat sink 336 is mounted on or proximate to an outer side 335 of each panel 317.

A method for disinfecting a person in a disinfection chamber assembly, e.g. disinfection chambers 100, 200 or 300, includes triggering at least one sensor, e.g. sensors 116, 216, or 316, in a disinfection chamber, e.g. disinfection chamber housing 102, 202, or 302. The method includes at least one of emitting a UV light with a UV light emitter, e.g. UV light emitters 214 or 314, or spraying a disinfection fluid from an atomization nozzle, e.g. atomization nozzle 114, for a stipulated duration. Emitting a UV light with the UV light emitter includes a emitting UV light with a plurality of UV light emitters. Emitting a UV light with the UV light emitter includes emitting light having a wavelength that ranges from 200 nm to 280 nm. Spraying the disinfection fluid includes starting a pump, e.g. pump 120, mounted above a disinfectant fluid tank, e.g. disinfectant fluid tank 118. The method includes stopping the emitting or the spraying after the stipulated duration. Stopping the spraying includes turning off the pump mounted above the disinfectant fluid tank. The method includes opening an exit door, e.g. door 126, to permit a person to exit the disinfection chamber housing after the stipulated duration. The method includes dissipating heat away from the UV light emitters with a heat sink, e.g. heat sink 236 or 336.

The methods and systems of the present disclosure, as described above and shown in the drawings, provide for disinfection chambers to kill viruses and other pathogens, while still meeting safety requirements. While the apparatus and methods of the subject disclosure have been shown and described with reference to preferred embodiments, those skilled in the art will readily appreciate that changes and/or modifications may be made thereto without departing from the scope of the invention as defined by the claims. Those skilled in the art will readily appreciate that a combination of both embodiments and/or other multi-stage ejector configurations could be used to meet various mission conditions in an optimal manner.

## Claims

1. A disinfection assembly for a person comprising:
a chamber housing (102) defining a floor area (104), an entry (106), an exit (108) and an interior space (110) between the entry and the exit;
at least one of a UV light emitter (314) or an atomization nozzle (114) positioned within the interior space; and
at least one platform (112) positioned and a sensor (116) operatively coupled to the platform.

2. The assembly as recited in claim 1, wherein the UV light emitter is configured and adapted to be turned on when a passenger is sensed on the platform.

3. The assembly as recited in claim 1 or 2, wherein the UV light emitter emits light having a wavelength that ranges from 200 nm to 280 nm.

4. The assembly as recited in any preceding claim, wherein the UV light emitter includes a plurality of UV light emitters.

5. The assembly as recited in any preceding claim, further comprising a heat sink in thermal communication with the UV light to dissipate heat therefrom.

6. The assembly as recited in any preceding claim, wherein the atomization nozzle is configured and adapted to be in fluid communication with a disinfection fluid tank (118), and optionally further comprising a pump (120) mounted above the disinfection fluid tank (118).

7. The assembly as recited in any preceding claim, wherein the atomization nozzle is configured and adapted to be turned on when a passenger is sensed on the platform.

8. The assembly as recited in any preceding claim, wherein the atomization nozzle includes a plurality of atomization nozzles, wherein each of the plurality of atomization nozzles is mounted to the chamber housing.

9. A method for disinfecting a person in a disinfection assembly:
triggering at least one sensor (116) in a disinfection chamber housing (102);
at least one of emitting a UV light with a UV light emitter (314), or spraying a disinfection fluid from an atomization nozzle (114) for a stipulated duration;
stopping at least one of the emitting or the spraying after the stipulated duration; and
opening an exit door (108) to permit a person to exit the disinfection chamber housing after the stipulated duration.

10. The method as recited in claim 9, wherein spraying the disinfection fluid includes starting a pump (120) mounted above a disinfectant fluid tank; and/or wherein stopping the spraying includes turning off a pump mounted above a disinfectant fluid tank (118).

11. The method as recited in claim 9 or 10, wherein emitting a UV light with the UV light emitter includes a emitting UV light with a plurality of UV light emitters.

12. The method as recited in claim 9, 10 or 11, wherein emitting a UV light with the UV light emitter includes emitting light having a wavelength that ranges from 200 nm to 280 nm.

13. The method as recited in any of claims 9 to 12, wherein the atomization nozzle includes a plurality of atomization nozzle, wherein each of the plurality of atomization nozzles is mounted to the chamber housing.

14. The method as recited in any of claims 9 to 13, wherein the atomization nozzle is configured and adapted to be in fluid communication with the disinfection fluid tank.

15. The method as recited in any of claims 9 to 14, further comprising dissipating heat away from the UV light emitter with a heat sink.
